# EUROPÄISCHE PATENTSCHRIFT

(11) **EP 1 153 086 B1**
(45) Veröffentlichungstag und Bekanntmachung des Hinweises auf die Patenterteilung: **27.04.2005**
(21) Anmeldenummer: 00902569.3
(22) Anmeldetag: 07.01.2000
(51) Int. Cl.: C09C 1/00

(54) **MEHRSCHICHTIGES PERLGLANZPIGMENT**
MULTILAYER NACREOUS PIGMENT
PIGMENT A LUSTRE NACRE MULTICOUCHE

(30) Priorität: 18.01.1999 DE 19901609
(43) Veröffentlichungstag der Anmeldung: 14.11.2001
(73) Patentinhaber: MERCK PATENT GmbH, 64293 Darmstadt (DE)
(72) Erfinder: ANDES, Stephanie, D-63452 Hanau (DE); FUCHS-POHL, Gerald, D-64331 Weiterstadt (DE); PFAFF, Gerhard, D-64839 Münster (DE)
(86) Internationale Anmeldenummer: PCT/EP2000/000071
(87) Internationale Veröffentlichungsnummer: WO 2000/042111

(56) Entgegenhaltungen:
- WO-A-93/08237
- DE-A- 19 618 569
- DE-A- 19 707 806

## Beschreibung

Die Erfindung betrifft ein mehrschichtiges Perlglanzpigment mit einem ausgeprägten Farbflop basierend auf einem plättchenförmigen Substrat aus einem Material mit niedriger Brechzahl im Bereich von 1,35 bis 1,8.

Mehrschichtige Pigmente, die einen winkelabhängigen Farbwechsel zwischen mehreren intensiven Interferenzfarben zeigen, sind bekannt.

So beschreibt US 4 434 010 ein mehrschichtiges Interferenzpigment, bestehend aus einer zentralen Schicht eines reflektierenden Materials (Aluminium) und alternierenden Schichten zweier transparenter, dielektrischer Materialien mit hoher und niedriger Brechzahl, beispielsweise Titandioxid und Siliziumdioxid beiderseits der zentralen Aluminiumschicht. In einer weiteren Ausführungsform des Pigmentes werden die auf die zentrale Aluminiumschicht folgenden Schichten durch Magnesiumfluorid und Chrom gebildet. Dieses Pigment zeigt einen intensiven Farbflop von grün nach purpurrot.

EP 0 753 545 beschreibt goniochromatische Glanzpigmente auf der Basis transparenter, nichtmetallischer, plättchenförmiger Substrate, die mindestens ein Schichtpaket aus einer farblosen Beschichtung mit einem Brechungsindex n ≤ 1,8 und einer reflektierenden, selektiv oder nichtselektiv absorbierenden, für sichtbares Licht zumindest teilweise durchlässigen Beschichtung sowie gewünschtenfalls zusätzlich eine äußere Schutzschicht aufweisen.

Diese Pigmente haben den Nachteil, daß sie durch einen technisch sehr aufwendigen und kostenintensiven Prozeß, beispielsweise durch Chemical Vapor Deposition (CVD)- oder Physical Vapor Deposition (PVD)-Verfahren hergestellt werden. Weiterhin nachteilig ist die oft schwere Reproduzierbarkeit der Pigmente in der gewünschten Produktqualität und ihre mangelnde Witterungsstabilität.

Aufgabe der vorliegenden Erfindung ist es, ein im wesentlichen transparentes Interferenzpigment mit kräftigen Interferenzfarben und/oder einer starken Winkelabhängigkeit der Interferenzfarben zur Verfügung zu stellen, das sich durch vorteilhafte Anwendungseigenschaften auszeichnet und gleichzeitig auf einfache Art und Weise hergestellt werden kann.

Diese Aufgabe wird gemäß der Erfindung gelöst durch ein mehrschichtiges Perlglanzpigment auf Basis eines plättchenförmigen Substrates aus einem Material mit niedriger Brechzahl im Bereich von 1,35 bis 1,8 das mindestens
(i) eine erste Schicht aus einem Material mit einer Brechzahl von größer als 1,8, sowie
(ii) eine semitransparente Metallschicht, die auf dem Substrat oder der Schicht (i) aufgebracht ist,
aufweist.

Wenn die semitransparente Metallschicht die äußere Schicht des Pigmentes bildet, können auch noch hoch- und niedrigbrechende Schichten sich anschließen. Bevor die Metallschicht aufgebracht wird, können sich die erste und zweite Schicht auch wiederholen.

Weiterhin wird diese Aufgabe gemäß der Erfindung gelöst durch ein Verfahren zur Herstellung des erfindungsgemäßen Pigmentes durch
- Aufbringen eines Precursors des Substratmaterials als dünner Film auf eine glatte Oberfläche,
- Verfestigung des flüssigen Filmes durch Trocknung,
- Ablösung des getrockneten Filmes und gegebenenfalls Behandlung mit einer Säure,
- Waschen der erhaltenen Substratpartikel und Resuspendierung in einer Beschichtungslösung,
- Beschichtung der Substratpartikel mit mindestens einer Schicht aus einem Material mit einer Brechzahl von größer als 1,8 sowie mindestens einer semitransparenten Metallschicht.

Die erfindungsgemäßen Pigmente können neben den rein koloristischen Anwendungen auch für funktionelle Anwendungen in Betracht gezogen werden. Beispiele hierfür sind Pigmente für den Sicherheitsbereich, z.B. Wert- und Sicherheitsdruck, Pigmente mit spezieller IR-Reflexion, z.B. für Gewächshausfolien und Pigmente für die Lasermarkierung von Kunststoffen.

Gegenstand der Erfindung ist weiterhin die Verwendung der erfindungsgemäßen Pigmente in Farben, Lacken, Druckfarben, Kunststoffen, keramischen Materialien, Gläsern und kosmetischen Formulierungen. Hierfür können sie auch als Mischungen mit handelsüblichen Pigmenten, beispielsweise anorganischen und organischen Absorptionspigmenten, Metalleffektpigmenten und LCP-Pigmenten, eingesetzt werden.

Die erfindungsgemäßen Pigmente basieren auf plättchenförmigen Substraten. Diese Substrate können aus Siliziumdioxid, Silikaten, Boroxid, Boraten, Aluminiumoxid, Glas, Magnesiumfluorid oder anderen transparenten, stabilen und zur Aufnahme von löslichen oder unlöslichen Farbmitteln befähigten Materialien bestehen.

Als Precursor für die Herstellung der Substrate werden Lösungen von anorganischen oder organischen Verbindungen der Metalle Aluminium, Silicium, Kalium oder Natrium mit Boraten, Aluminaten, Poly- bzw. Metaphosphaten, Silikaten oder Gemischen derselben eingesetzt. Ein bevorzugter Precursor ist Wasserglas.

Die plättchenförmigen Substratpartikel haben eine Dicke zwischen 0,05 und 5 µm und insbesondere zwischen 0,2 und 2 µm. Die Ausdehnung in den beiden anderen Dimensionen beträgt zwischen 1 und 250 µm und insbesondere zwischen 2 und 100 µm.

In das Substrat können als unlösliche Farbmittel Pigmentpartikel eingelagert sein, deren Abmessungen deutlich geringer sind als die Dicke des Substrates. Die Partikelgröße der handelsüblichen Pigmente muß deshalb an die gewünschte Schichtdicke des Substrates angepaßt werden. Der Begriff Pigmentpartikel ist hier weit zu fassen und umfaßt Weiß-, Schwarz-, Bunt- sowie Leuchtpigmente.

Geeignete anorganische Pigmente sind Weißpigmente wie Titandioxid, Bariumsulfat oder Zinkoxid, beispielsweise Titandioxid 2310 (Hersteller: Kronos), Titandioxid R-D (Hersteller: Bayer), Titandioxid R-506 (Hersteller: Sachtleben).

Geeignete Schwarzpigmente sind Magnetit oder Pigmentruß, beispielsweise Farbruß FW 200 (Degussa).

Als Buntpigmente sind Eisen- oder Chromoxid, Mischphasenoxide wie (Ti, Cr, Sb)O₂, CoAl₂O₄ (Thenards Blau), ZnAl₂O₄ (Rinmans Grün), (Fe, Cr)₂O₃ sowie Sulfide, beispielsweise CdS, geeignet.

Geeignet sind auch anorganische Leuchtpigmente, wie fluoreszierendes silberdotiertes Zinkoxid, phosphoreszierendes kupferdotiertes Zinksulfid oder Ultramarinpigmente.

Geeignete organische Pigmente sind Azopigmente, Anthrachinon-Pigmente, Indigo oder Thioindigo-Derivate, Diketo-Pyrrolo-Pyrrol-Pigmente, Perylen-Pigmente oder Phthalocyanin-Pigmente. Besonders geeignete Rotpigmente sind Paliogen Maron L3920 (Hersteller: BASF), DPP-Irgazin Rot BO (Hersteller: Ciba), Chinquaisia Margenta RT355D (Hersteller: Ciba), Hostaperm Rot E2B70 (Hersteller: Hoechst - Clariant), Sicotrans Rot L2817 (Hersteller: BASF), Carmin Rot, Thioindigo, DC Rot 6 auch als Lithol Rubin 13 bezeichnet, DC Rot 33 auch als Acid Fuchsine bezeichnet. Besonders geeignete Blaupigmente sind Hostaperm Blau AFL (Hersteller: Hoechst - Clariant), Irgazin Blau A3RN (Hersteller: Ciba), Paliogen Blau L6470 (Hersteller: BASF), Berliner Blau, FDC Blau 1 auch als Brilliant Blau bezeichnet. Besonders geeignete Grünpigmente sind Monastral Grün 64 Spezial (Hersteller: Zeneca - ICI), Hostaperm Grün 8G (Hersteller: Hoechst - Clariant), DC Grün 5 auch als Alizarin Cyanin Grün F bezeichnet und als Gelbpigmente sind besonders geeignet Irgazin Gelb 5GTL (Hersteller: Ciba), Irgacolor Gelb 2GLMA (Hersteller: Ciba), FDC Gelb 5 auch als Tartrazin bezeichnet, FDC Gelb 6 auch als Sunset Gelb bezeichnet.

In vielen Fällen ist es vorteilhaft, zur besseren Dispergierung der Pigmentpartikel im Precursor noch Netzmittel zuzusetzen, beispielsweise Hydropallat 884 (Hersteller: Henkel). Weder der Typ noch die Menge des zugesetzten Netzmittels sind kritisch, aber im allgemeinen liegt der Anteil des Netzmittels maximal bei 2 Gew.-%, bezogen auf die Dispersion.

Der auf das Gewicht des unbeschichteten Substrates bezogene Gewichtsanteil der eingelagerten Pigmentpartikel liegt zwischen 0,5 und 40 % und insbesondere zwischen 5 und 25 %. Weitere Angaben sind der Europäischen Patentschrift 0 608 388 zu entnehmen.

Das Substrat kann als Farbmittel aber auch ein lösliches Farbmittel enthalten. Unter dem Begriff "lösliches Farbmittel" ist entweder ein farbgebendes Metalloxid, beispielsweise Eisenoxid, Chromoxid oder Kobaltoxid, oder ein löslicher organischer Farbstoff zu verstehen.

Generell sind für die Anfärbung des Substrates farbgebende Verbindungen der Metalle Titan, Vanadium, Chrom, Mangan, Eisen, Kobalt, Nickel und Kupfer, vorzugsweise Verbindungen von Kobalt, Kupfer, Eisen und Chrom, geeignet. Sie werden als lösliche Verbindungen dem Precursor des Substrates zugesetzt. Man erhält ein farbiges, transparentes Substrat mit einer Farbskala, ähnlich derjenigen von farbigem, transparenten Glas. Durch Zusatz von Eisenverbindungen erhält man zum Beispiel rotbraune Farbtöne, durch Zusatz von Chromverbindungen grüne Farbtöne und durch Zusatz von Kobaltverbindungen blaue Farbtöne.

Als lösliche organische Farbstoffe können in Lauge lösliche Hydroxyanthrachinonfarbstoffe oder saure Azofarbstoffe eingesetzt werden.

Das lösliche Farbmittel ist im unbeschichteten Substrat in einem Anteil von 0,01 bis 50 Gew.-%, vorzugsweise 1 bis 30 Gew.-% enthalten. Weitere Angaben sind EP 0 608 388 zu entnehmen.

Als Schichtmaterial für die Schicht (i) mit einer Brechzahl von größer als 1,8 eignen sich alle dem Fachmann bekannten hochbrechenden Materialien, die filmartig und dauerhaft auf die Substratteilchen aufgebracht werden können. Besonders geeignet sind Metalloxide oder Metalloxidgemische, wie z.B. TiO₂, Fe₂O₃, ZrO₂, ZnO, SnO₂, oder Verbindungen mit hoher Brechzahl, wie z.B. Eisentitanate, Eisenoxidhydrate, Titansuboxide, Chromoxid, Bismutvanadat, Cobaltaluminat sowie Mischungen bzw. Mischphasen der genannten Verbindungen untereinander oder mit anderen Metalloxiden. Es kommen auch Metallsulfide, Metallnitride oder Metalloxynitride in Frage.

Die Dicke der Schicht (i) beträgt 10 - 550 nm, vorzugsweise 15 - 400 nm und insbesondere 20 - 350 nm.

Geeignete Schichtmaterialien für die Schicht mit niedriger Brechzahl (iii) sind vorzugsweise Metalloxide bzw. die entsprechenden Oxidhydrate, wie z.B. SiO₂, Al₂O₃, AIO(OH), B₂O₃ oder ein Gemisch der genannten Metalloxide oder MgF₂. Die Dicke der Schicht beträgt 10 - 1000 nm, vorzugsweise 20 - 800 nm und insbesondere 30 - 600 nm.

Als Material mit niedriger Brechzahl können jedoch auch Polymere eingesetzt werden, wie z.B. Acrylate. Die verwendeten Monomere haben ein Molekulargewicht von 200 bis 1000 und stehen als Mono-, Di- oder Triacrylate zur Verfügung. Hinsichtlich funktioneller Gruppen sind sie als Kohlenwasserstoffe, Polyole, Polyether, Silicone oder als fluorierte teflonartige Monomere verfügbar. Diese Monomere können durch Elektronenstrahlen oder UV-Strahlen polymerisiert werden. Die erhaltenen Schichten besitzen eine Temperaturstabilität bis zu 250 °C. Die Brechzahlen der Acrylatschichten liegen im Bereich von 1,35 bis 1,60. Weitere Angaben sind bei David G. Shaw und Marc G. Langlois zu finden: Use of a new high speed acrylate deposition process to make novel multilayer structures, MRS-Tagung in San Francisco 1995; A new high speed process for vapor depositing fluoro and silicone acrylates for release coating applications, Tagung der Society of Vacuum Coater in Chicago, Illinois, 1995.

Die Schichtdicke der Polymerschicht wird auf Werte zwischen 20 und 700 nm, bevorzugt zwischen 60 und 500 nm eingestellt.

Die Metallschichten (ii) bestehen aus Metallen, wie z.B. Aluminium, Chrom, Nickel, Chrom-Nickel-Legierungen oder Silber. Chrom und Aluminium sind dabei bevorzugt, da sie leicht abzuscheiden sind. Die Schichtdicke der Metallschichten wird auf 5 bis 20 nm eingestellt, um Semitransparenz zu erhalten. Als semitransparente Reflektorschichten können aber auch Materialien wie Graphit oder Titannitrid eingesetzt werden.

Die erfindungsgemäßen Pigmente enthalten auch in der Metalloxid-Beschichtung zusätzliche Farbmittel. Werden beispielsweise Rußpartikel verwendet, dann werden Partikelgrößen von 5 bis 200 nm und insbesondere 10 bis 100 nm verwendet. Derartige Pigmente, die bevorzugt Rußpartikel in Schichten aus Titandioxid, Eisenoxid, Zinnoxid, Chromoxid und Zinkoxid enthalten sind in EP 0 499 864 beschrieben.

Weiterhin können die erfindungsgemäßen Pigmente auch Partikel aus Titandioxid, Aluminiumoxid, Siliciumdioxid, Zinndioxid, Magnesiumoxid, Zinkoxid, Cerdioxid, Wolframoxid, Molybdänoxid, Zirkonoxid, aber auch Mischoxide, wie Cr₂FeO₄, CoAl₂O₄ oder NiAl₂O₄, in der Metalloxidschicht enthalten.

Anstelle anorganischer Pigmentpartikel können auch organische Pigmentpartikel in der Metalloxidschicht enthalten sein, wobei insbesondere temperaturstabile organische Pigmente bevorzugt sind. Als organische Pigmentpartikel werden vorzugsweise Phthalocyanine, Verlackungsprodukte von basischen Farbstoffen mit Heteropolysäuren, Anthrachinone, Phenazine, Phenoxazine, Diketopyrrolopyrrole oder Perylene verwendet. Prinzipiell können alle Pigmente, die für die Einarbeitung in das Substrat beschrieben wurden auch in die Beschichtung des erfindungsgemäßen Pigmentes eingearbeitet werden. Die Einlagerung von kleinen Metalloxid- oder organischen Pigmentpartikeln mit einer durchschnittlichen Größe von 10 bis 40 nm in die Hohlräume der Metalloxidbeschichtung bewirkt eine deutliche Erhöhung des Deckvermögens und des Glanzes, verbunden mit einer hohen Homogenität der Beschichtung im Vergleich zu Pigmenten, die durch Mischfällung erhalten werden. Das Deckvermögen und bei gefärbten Pigmentpartikeln die beobachtungswinkelabhängige Absorptionsfarbe der erfindungsgemäßen Pigmente kann in einem weiten Bereich durch die Konzentration der eingelagerten Pigmentpartikel variiert werden. Der auf die Beschichtung bezogene Massenanteil der eingelagerten Pigmentpartikel liegt zwischen 0,5 und 30 % und insbesondere zwischen 2 und 20 %. Weitere Angaben zu Pigmenten, die in der Beschichtung Pigmentpartikel enthalten, sind DE 41 40 295 zu entnehmen.

Das fertige Pigment wird einer Nachbeschichtung oder Nachbehandlung (iv) unterzogen, die die Licht-, Wetter- und chemische Stabilität weiter erhöht, oder die Handhabung des Pigments, insbesondere die Einarbeitung in unterschiedliche Medien, erleichtert. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage.

Die zusätzlich aufgebrachten Stoffe machen nur etwa 0,1 bis 5 Gew.-%, vorzugsweise etwa 0,5 bis 3 Gew.-%, des gesamten Pigmentes aus.

Die Anzahl und Dicke der Schichten ist abhängig vom gewünschten Effekt und dem verwendeten Substrat. Die Anzahl der Schichten wird durch die Wirtschaftlichkeit des Pigmentes begrenzt. Verwendet man als Substrat SiO₂-Plättchen, die nach dem in EP 0 608 388 beschriebenen Verfahren auf einem endlosen Band hergestellt werden, lassen sich besonders gut definierte Interterenzeffekte erzielen, da im Gegensatz zu Glimmer diese SiO₂-Plättchen eine einheitliche Schichtdicke besitzen. Das Reflexions- bzw. Transmissionsspektrum eines solchen Pigmentes weist feinere und genauer abstimmbare Strukturen auf als das Spektrum eines entsprechenden Pigmentes, das auf einem Substrat mit breiter Dickenverteilung, wie z.B. Glimmer, beruht.

Die SiO₂-Plättchen werden gemäß der EP 0 608 388 auf einem endlosen Band durch Verfestigung und Hydrolyse einer Wasserglaslösung hergestellt.

Die Metalloxidschichten werden vorzugsweise naßchemisch aufgebracht, wobei die zur Herstellung von Perlglanzpigmenten entwickelten naßchemischen Beschichtungsverfahren angewendet werden können; derartige Verfahren sind z.B. beschrieben in DE 14 67 468, DE 19 59 988, DE 20 09 566, DE 22 14 545, DE 22 15 191, DE 22 44 298, DE 2313 331, DE 25 22 572, DE 31 37 808, DE 31 37 809, DE 31 51 343, DE 31 51 354, DE 31 51 355, DE 32 11 602, oder DE 32 35 017.

Zur Beschichtung werden die Substratpartikel in Wasser suspendiert und mit einem oder mehreren hydrolysierbaren Metallsalzen bei einem für die Hydrolyse geeigneten pH-Wert versetzt, der so gewählt wird, daß die Metalloxide bzw. Metalloxidhydrate direkt auf den Partikeln niedergeschlagen werden, ohne daß es zu Nebenfällungen kommt Der pH-Wert wird üblicherweise durch gleichzeitiges Zudosieren einer Base konstant gehalten. Anschließend werden die Pigmente abgetrennt, gewaschen und getrocknet und gegebenenfalls geglüht, wobei die Glühtemperatur im Hinblick auf die jeweils vorliegende Beschichtung optimiert werden kann. Falls gewünscht können die Pigmente nach Aufbringen einzelner Beschichtungen abgetrennt, getrocknet und ggf. geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Periglanzpigmenten vorgeschlagenen Verfahren entsprechend angewendet werden können.

Für das Aufbringen von Titandioxidschichten wird das im US 3 553 001 beschriebene Verfahren bevorzugt.

Zu einer auf etwa 50-100 °C, insbesondere 70-80 °C erhitzten Suspension des zu beschichtenden Materials wird langsam eine wäßrige Titansalzlösung zugegeben, und es wird durch gleichzeitiges Zudosieren einer Base, wie z.B. wäßrige Ammoniaklösung oder wäßrige Alkalilauge, ein weitgehend konstanter pH-Wert von etwa 0,5-5, insbesondere etwa 1,5-2,5 eingehalten. Sobald die gewünschte Schichtdicke der TiO₂-Fällung erreicht ist, wird die Zugabe der Titansalzlösung und der Base gestoppt.

Dieses, auch als Titrationsverfahren bezeichnete Verfahren zeichnet sich dadurch aus, daß ein Überschuß an Titansalz vermieden wird. Das wird dadurch erreicht, daß man pro Zeiteinheit nur eine solche Menge der Hydrolyse zuführt, wie sie für eine gleichmäßige Beschichtung mit dem hydratisierten TiO₂ erforderlich ist und wie pro Zeiteinheit von der verfügbaren Oberfläche aufgenommen werden kann. Es entstehen deshalb keine hydratisierten Titandioxidteilchen, die nicht auf der zu beschichtenden Oberfläche niedergeschlagen sind.

Für das Aufbringen der Siliziumdioxidschichten ist folgendes Verfahren anzuwenden: Zu einer auf etwa 50-100 °C, insbesondere 70-80 °C erhitzten Suspension des zu beschichtenden Materials wird eine Natronwasserglaslösung zudosiert. Durch gleichzeitige Zugabe von 10%iger Salzsäure wird der pH-Wert bei 4 bis 10, vorzugsweise bei 6,5 bis 8,5 konstant gehalten. Nach Zugabe der Wasserglaslösung wird noch 30 min nachgerührt.

Die einzelnen Schichten können auch nach bekannten Verfahren durch Sputtern von Metallen, beispielsweise von Aluminium oder Chrom oder von Legierungen, wie zum Beispiel Cr-Ni-Legierungen sowie von Metalloxide, beispielsweise von Titanoxid, Siliciumoxid, oder Indium-Zinn-Oxid oder durch thermisches Verdampfen von Metallen, Metalloxiden oder Acrylaten hergestellt werden. Bevorzugt wird eine Vakuumbandbeschichtung, wie sie in DE 197 07 805 und in DE 197 07 806 für die Herstellung von Interferenzpigmenten beschrieben wird.

## Patentansprüche

1. Mehrschichtiges Perlglanzpigment auf Basis eines plättchenförmigen Substrates aus einem Metall mit niedriger Brechzahl im Bereich von 1,35 bis 1,8, das mindestens
(i) eine erste Schicht aus einem Material mit einer Brechzahl von größer als 1,8 sowie
(ii) eine semitransparente Metallschicht, die auf dem Substrat oder der Schicht (i) aufgebracht ist,
aufweist.

2. Mehrschichtiges Perlglanzpigment nach Anspruch 1, **dadurch gekennzeichnet, daß** es zusätzlich mindestens
(iii) eine zweite Schicht aus einem Material mit niedriger Brechzahl im Bereich von 1,35 bis 1,8 enthält und die semitransparente Metallschicht auf dem Substrat oder den Schichten (i) oder (iii) aufgebracht ist.

3. Mehrschichtiges Perlglanzpigment nach Anspruch 1 oder 2, **dadurch gekennzeichnet, daß** es eine Nachbeschichtung (iv) aufweist.

4. Perlglanzpigment nach Anspruch 1, **dadurch gekennzeichnet, daß** das Substrat plättchenförmiges Siliciumdioxid, Aluminiumoxid, Boroxid oder Magnesiumfluorid ist.

5. Perlglanzpigment nach mindestens einem der Ansprüche 1 bis 4, **dadurch gekennzeichnet, daß** das Material mit hoher Brechzahl TiO₂, ZrO₂, Fe₂O₃, Fe₃O₄, Cr₂O₃, ZnO oder ein Gemisch aus diesen Oxiden oder ein Eisentitanat, ein Eisenoxidhydrat, ein Titansuboxid oder eine Mischung bzw. Mischphase dieser Verbindungen ist.

6. Perlglanzpigment nach mindestens einem der Ansprüche 1 bis 5, **dadurch gekennzeichnet, daß** das Material mit niedriger Brechzahl SiO₂, Al₂O₃, AlOOH, B₂O₃, MgF₂ oder ein Acrylat ist, wobei gegebenenfalls Alkali- oder Erdalkalioxide als zusätzliche Bestandteile enthalten sein können.

7. Verfahren zur Herstellung eines mehrschichtigen Perlglanzpigmentes gemäß den Ansprüchen 1-6 durch
- Aufbringen eines Precursors des Substratmaterials als dünner Film auf ein endloses Band,
- Verfestigung des flüssigen Films durch Trocknung,
- Ablösung des getrockneten Films und gegebenenfalls Behandlung mit einer Säure,
- Waschen der erhaltenen Substratpartikel und Resuspendierung in einer Beschichtungslösung,
- Beschichtung der Substratpartikel mit mindestens einer Schicht aus einem Material mit einer Brechzahl von größer als 1,8 sowie mindestens einer semitransparenten Metallschicht.

8. Verfahren nach Anspruch 7, **dadurch gekennzeichnet, daß** zusätzlich mindestens eine Schicht aus einem Material mit niedriger Brechzahl im Bereich von 1,35 bis 1,8 aufgebracht wird.

9. Verfahren nach einem der Ansprüche 7 oder 8, **dadurch gekennzeichnet, daß** zusätzlich eine Nachbeschichtung aufgebracht wird.

10. Verfahren nach mindestens einem der Ansprüche 7 bis 9, **dadurch gekennzeichnet, daß** als Precursor Lösungen von anorganischen oder organischen Verbindungen der Metalle Aluminium, Silicium, Kalium oder Natrium mit Boraten, Chloride, Aluminaten, Poly- bzw. Metaphosphaten, Silikaten oder Gemischen derselben eingesetzt werden.

11. Verfahren nach mindestens einem der Ansprüche 7 bis 10, **dadurch gekennzeichnet, daß** der Precursor Wasserglas ist.

12. Verfahren nach mindestens einem der Ansprüche 7 - 11, **dadurch gekennzeichnet, daß** dem Precursor Farbmittel zugesetzt werden, wobei das Farbmittel entweder vor dem Auftragen auf das Band im Precursor dispergiert oder gelöst oder die Komponenten über mehrere Düsen getrennt auf das Band aufgetragen werden.

13. Verfahren nach mindestens einem der Ansprüche 7 - 12, **dadurch gekennzeichnet, daß** vor dem Aufbringen oder beim Aufbringen auf das endlose Band feine Partikel eines organischen oder anorganischen Pigmentes im Precursor dispergiert werden.

14. Verfahren nach Anspruch 13, **dadurch gekennzeichnet, daß** die dispergierte Menge an Pigmentpartikeln 0,01 bis 99 Gew.-%, vorzugsweise 1 bis 30 Gew.-%, bezogen auf den Precursor beträgt.

15. Verfahren nach mindestens einem der Ansprüche 7 - 14, **dadurch gekennzeichnet, daß** die Schichten nach Zwischentrocknung des zu beschichtenden Materials in einem Wirbelbettreaktor durch CVD und/oder PVD aufgebracht werden.

16. Verwendung der Pigmente nach den Ansprüchen 1 bis 6 zur Pigmentierung von Lacken, Druckfarben, Kunststoffen, Kosmetika, Glasuren für Keramiken und Gläser.

17. Verwendung der Pigmente nach den Ansprüchen 1 bis 6 zur Lasermarkierung von Kunststoffen.

18. Lacke, Druckfarben, Kunststoffe, Kosmetika, Keramiken, Gläser und Polymerfolien, welche mit einem Pigment nach den Ansprüchen 1 bis 6 pigmentiert sind.

19. Lasermarkierbare Kunststoffe enthaltend Pigmente nach den Ansprüchen 1 bis 6.

## Claims

1. Multilayer pearl lustre pigment on the basis of a platelet-shaped substrate comprising a material with low refractive index in the range from 1.35 to 1.8, which comprises at least
(i) a first layer of a material having a refractive index of more than 1.8 and
(ii) a semitransparent metal layer which is applied to the substrate or to the layer (i).

2. Multilayer pearl lustre pigment according to Claim 1, **characterized in that** it further comprises at least
(iii) a second layer of a material with low refractive index in the range from 1.35 to 1.8, and the semitransparent metal layer is applied to the substrate or to the layers (i) or (iii).

3. Multilayer pearl lustre pigment according to Claim 1 or 2, **characterized in that** it has an aftercoating (iv).

4. Pearl lustre pigment according to Claim 1, **characterized in that** the substrate is platelet-shaped silicon dioxide, aluminium oxide, boron oxide or magnesium fluoride.

5. Pearl lustre pigment according to at least one of Claims 1 to 4, **characterized in that** the material of high refractive index is TiO₂, ZrO₂, Fe₂O₃, Fe₃O₄, Cr₂O₃, ZnO or a mixture of these oxides or an iron titanate, an iron oxide hydrate, a titanium suboxide or a mixture and/or mixed phase of these compounds.

6. Pearl lustre pigment according to at least one of Claims 1 to 5, **characterized in that** the material with low refractive index is SiO₂, Al₂O₃, AlOOH, B₂O₃, MgF₂ or an acrylate, it being possible if desired for alkali metal oxides or alkaline earth metal oxides to be present as additional constituents.

7. Process for producing a multilayer pearl lustre pigment according to Claims 1 to 6 by
- applying a precursor of the substrate material as a thin film to a continuous belt,
- solidifying the liquid film by drying,
- detaching the dried film and optionally treating with an acid,
- washing the resultant substrate particles and resuspending them in a coating solution,
- coating the substrate particles with at least one layer of a material having a refractive index of more than 1.8 and at least one semitransparent metal layer.

8. Process according to Claim 7, **characterized in that** additionally at least one layer of a material with low refractive index in the range from 1.35 to 1.8 is applied.

9. Process according to one of Claims 7 or 8, **characterized in that** additionally an aftercoating is applied.

10. Process according to at least one of Claims 7 to 9, **characterized in that** solutions of organic or inorganic compounds of the metals aluminium, silicon, potassium or sodium with borates, chlorides, aluminates, polyphosphates and/or metaphosphates, silicates or mixtures thereof are used as precursor.

11. Process according to at least one of Claims 7 to 10, **characterized in that** the precursor is waterglass.

12. Process according to at least one of Claims 7-11, **characterized in that** colorants are added to the precursor, either the colorant being dissolved or dispersed in the precursor before application to the belt or the components being applied to the belt separately via two or more nozzles.

13. Process according to at least one of Claims 7-12, **characterized in that** before or during application to the continuous belt fine particles of an organic or inorganic pigment are dispersed in the precursor.

14. Process according to Claim 13, **characterized in that** the dispersed amount of pigment particles is from 0.01 to 99% by weight, preferably from 1 to 30% by weight, based on the precursor.

15. Process according to at least one of Claims 7-14, **characterized in that** following drying of the material to be coated the layers are applied in a fluidized-bed reactor by CVD and/or PVD.

16. Use of the pigments according to Claims 1 to 6 for pigmenting paints, printing inks, plastics, cosmetics, glazes for ceramics, and glasses.

17. Use of the pigments according to Claims 1 to 6 for the laser marking of plastics.

18. Paints, printing inks, plastics, cosmetics, ceramics, glasses and polymer films pigmented with a pigment according to Claims 1 to 6.

19. Laser-markable plastics comprising pigments according to Claims 1 to 6.

## Revendications

1. Pigment nacré multicouche à base d'un substrat en forme de paillettes fait d'une matière ayant un faible indice de réfraction de 1,35 à 1,8, qui présente au moins
(i) une première couche faite d'une matière ayant un indice de réfraction supérieur à 1,8 et
(ii) une couche métallique semitransparente déposée sur le substrat ou sur la couche (i).

2. Pigment nacré multicouche selon la revendication 1, **caractérisé en ce qu'**il contient en outre au moins
(iii) une deuxième couche faite d'une matière ayant un indice de réfraction plus faible de 1,35 à 1,8 et **en ce que** la couche métallique semitransparente est déposée sur le substrat ou sur la couche (i) ou (iii).

3. Pigment nacré multicouche selon la revendication 1 ou 2, **caractérisé en ce qu'**il présente un revêtement supplémentaire (iv).

4. Pigment nacré multicouche selon la revendication 1, **caractérisé en ce que** le substrat est fait de dioxyde de silicium, d'oxyde d'aluminium, d'oxyde de bore ou de fluorure de magnésium en forme de paillettes.

5. Pigment nacré multicouche selon au moins l'une des revendications 1 à 4, **caractérisé en ce que** la matière ayant un indice de réfraction élevé est TiO₂, ZrO₂, Fe₂O₃, Fe₃O₄, Cr₂O₃, ZnO ou un mélange de ces oxydes ou un titanate de fer, un oxyde hydraté de fer, un sous-oxyde de titane ou un mélange ou une phase mixte de ces composés.

6. Pigment nacré multicouche selon au moins l'une des revendications 1 à 5, **caractérisé en ce que** la matière ayant un faible indice de réfraction est SiO₂, Al₂O₃, AlOOH, B₂O₃, MgF₂ ou un acrylate, dans lequel des oxydes de métaux alcalins ou alcalino-terreux peuvent éventuellement être contenus en tant que composants supplémentaires.

7. Procédé de production d'un pigment nacré multicouche selon les revendications 1 à 6, consistant à :
- déposer un précurseur de la matière de substrat sous forme de film mince sur une bande sans fin,
- durcir le film liquide au moyen d'un séchage,
- enlever le film séché et le traiter éventuellement avec un acide,
- laver les particules de substrat obtenues et les remettre en suspension dans une solution de revêtement,
- revêtir les particules de substrat avec au moins une couche d'une matière ayant un indice de réfraction supérieur à 1,8 ainsi qu'avec au moins une couche métallique semitransparente.

8. Procédé selon la revendication 7, **caractérisé en ce que** l'on dépose en plus au moins une couche d'une matière ayant un faible indice de réfraction de 1,35 à 1,8.

9. Procédé selon l'une des revendications 7 ou 8, **caractérisé en ce que** l'on dépose en plus un revêtement supplémentaire.

10. Procédé selon au moins l'une des revendications 7 à 9, **caractérisé en ce que** l'on met en oeuvre, en tant que précurseur, des solutions de composés inorganiques ou organiques des métaux aluminium, silicium, potassium ou sodium avec des borates, des chlorures, des aluminates, des poly- ou des méta-phosphates, des silicates ou des mélanges de ceux-ci.

11. Procédé selon au moins l'une des revendications 7 à 10, **caractérisé en ce que** le précurseur est du silicate de potassium.

12. Procédé selon au moins l'une des revendications 7 à 11, **caractérisé en ce que** l'on ajoute un colorant au précurseur, le colorant étant dispersé ou dissous dans le précurseur avant le dépôt sur la bande ou les composants étant déposés séparément sur la bande par l'intermédiaire de plusieurs buses.

13. Procédé selon au moins l'une des revendications 7 à 12, **caractérisé en ce que** l'on disperse de fines particules d'un pigment organique ou inorganique dans le précurseur avant le dépôt ou pendant le dépôt sur la bande sans fin.

14. Procédé selon la revendication 13, **caractérisé en ce que** la proportion de particules de pigment dispersées va de 0,01% à 99% en poids, de préférence de 1% à 30% en poids par rapport au précurseur.

15. Procédé selon au moins l'une des revendications 7 à 14, **caractérisé en ce que** les couches sont déposées, après séchage intermédiaire de la matière à revêtir, dans un réacteur à lit fluidisé au moyen d'un procédé de dépôt chimique en phase vapeur (CVD) et/ou d'un procédé de dépôt physique en phase vapeur (PVD).

16. Utilisation des pigments selon les revendications 1 à 6 pour pigmenter des vernis, des encres d'impression, des matières plastiques, des produits cosmétiques, des glaçures pour céramiques et des verres.

17. Utilisation des pigments selon les revendications 1 à 6 pour le marquage au laser de matières plastiques.

18. Vernis, encres d'impression, matières plastiques, produits cosmétiques, céramiques, verres et feuilles de polymère, lesquels ont été pigmentés avec un pigment selon les revendications 1 à 6.

19. Matières plastiques marquables au laser contenant des pigments selon les revendications 1 à 6.
